# EUROPEAN PATENT SPECIFICATION

(11) **EP 1 272 495 B1**
(45) Date of publication and mention of the grant of the patent: **30.06.2004**
(21) Application number: 01940312.0
(22) Date of filing: 10.04.2001
(51) Int. Cl.: C07D 501/36

(54) **A PROCESS FOR THE PREPARATION OF HIGHLY CRYSTALLINE SODIUM CEFOPERAZONE**
VERFAHREN ZUR HERSTELLUNG VON HOCHKRISTALLINEM NATRIUM CEFOPERAZON
PROCEDE DE PREPARATION DE CEFOPERAZONE SODIQUE HAUTEMENT CRISTALLINE

(30) Priority: 14.04.2000 IT MI000837
(43) Date of publication of application: 08.01.2003
(73) Proprietor: Antibioticos S.p.A., 20090 Rodano (IT)
(72) Inventor: CABRI, Walter, I-20089 Rozzano (IT); POZZI, Giovanni, I-20045 Besana Brianza (IT); GHETTI, Paolo, I-20090 Segrate (IT); VERGANI, Domenico, I-20058 Villasanta (IT); STRIGARO, Roberto, I-20155 Milano (IT)
(74) Representative: Minoja, Fabrizio, Dr.
(86) International application number: PCT/EP2001/004074
(87) International publication number: WO 2001/079210

(56) References cited:
- GB-A- 1 508 071
- US-A- 4 456 753

## Description

The present invention relates to a process for the preparation of sterile, highly crystalline sodium Cefoperazone 7-[[[[(4-ethyl-2,3-dioxo-1-piperazinyl)carbonyl]amino](4-hydroxyphenyl)-acetyl]-amino]-3-[[(1-methyl-1H-tetrazol-5-yl)thio]methyl]-8-oxo5-thia-1-azabicyclo[4.2.0]oct-2-ene-2-carboxylic acid, monosodium salt, [6R-[6α,7β-(R*)]]), in the form of needle microcrystal agglomerates which are a further object of the invention.

Sodium Cefoperazone, disclosed in US 4,087,424 (5/1978), is an injectable semi-synthetic cephalosporin with broad spectrum activity against resistant Gram negative bacteria, widely used in clinic due to its effectiveness in the cases of severe infections of skin, high and lower respiratory tract, central nervous system and genital-urinary system.

Sterile sodium Cefoperazone is available both in the amorphous (GB 1,508,071) and crystalline form (US 4,458,070, 07/1982). The crystalline product, obtained by crystallization, has higher purity, higher chemical stability, and lower hygroscopicity than the amorphous product, usually obtained by freeze-drying. The preparation of the product in the crystalline form is disclosed in US 4,458,070 and in US 4,456,753: US 4,458,070, (07/1982) comprises the crystallization of sodium Cefoperazone from a water/acetone/methylene chloride mixture, whereas US 4,456,753 (02/1983) discloses an improved process providing a "highly crystalline" product from the acetone/water mixture, thus avoiding the use of a toxic solvent such as methylene chloride (class 1, ICH guidelines). This method, however, provides highly crystalline sodium Cefoperazone in the form of free needle microcrystals. It is therefore difficult to filter or centrifugate the resulting suspension with the apparatuses usually employed for the preparation of sterile bulk, due to the compressibility of the product and its tendency to pack. As a consequence, drying is carried out with detriment of the process productivity and costs.

### SUMMARY OF THE INVENTION

We have now surprisingly found a process for the preparation of Cefoperazone in the form of needle microcrystal agglomerates, which are easy to filter and to dry, by crystallization of the sodium salt from water/acetone mixtures in the presence of alcohols and/or mixtures thereof. The process of the present invention is therefore characterized by high productivity on the industrial scale. The resulting sodium Cefoperazone is still as highly crystalline as that disclosed in US 4,456,753.

### DETAILED DISCLOSURE OF THE INVENTION

The process of the present invention comprises the salification of acid Cefoperazone in water/acetone mixtures with one equivalent of a base selected from sodium bicarbonate, sodium carbonate and sodium 2-ethylhexanoate, keeping the temperature at 5 to 25°C. The sodium Cefoperazone solution is added with C₁-C₄ aliphatic alcohols or mixtures thereof at temperatures of 20°C to 40°C. Cefoperazone sodium is crystallized by addition of acetone, keeping the temperature at 20 to 40°C; said addition is preferably carried out in two portions: the first portion is sufficient to form a sodium Cefoperazone suspension, the second portion allows to complete the crystallization to a low content in sodium Cefoperazone in the mother liquors. The filtration or centrifugation of the product can be carried out after 90 minutes or one day after the end of the crystallization, at the crystallization temperature (20 to 40°C) or after cooling the suspension to temperatures of 0 to 20°C. The crystalline product is dried under vacuum (20 to 300 mbars) adjusting the temperature of the product to 35 - 40°C.

Acid Cefoperazone is salified by using preferably water/acetone/Cefoperazone ratios of 0.8-0.6/1,4-5,0/1,0 v/v/w with one equivalent of a base, preferably sodium bicarbonate, in amounts of 0.124∼0.130 grams/ 1 gram of acid Cefoperazone, thereby obtaining water/acetone/sodium Cefoperazone solutions containing 19 ∼ 35% of water at a temperature of 18∼22°C. The sodium Cefoperazone solution is added with alcohols or mixtures thereof selected from methanol, ethanol, n-propanol, n-butanol and isopropanol at temperatures of 20°C to 40°C, preferably at 23 to 30°C. Ethanol (0.2-0.4 ml per 1 gram of acid Cefoperazone) or isopropanol (0.2-0.8 ml per 1 gram of acid Cefoperazone) or mixtures thereof (ethanol/isopropanol: 0.2-0.4/0.2-0.8 ml per 1 gram of acid Cefoperazone) are preferably used. Sodium Cefoperazone is crystallized by adding acetone in two portions, keeping the temperature at 20 to 40°C, preferably at 23 to 30°C. The first acetone portion, sufficient to form a Cefoperazone sodium suspension, provides a water/acetone mixture containing 11 to 14% of water. The second portion provides complete crystallization which is attained when the water/acetone mixture has water content of 3 to 5%. The product may be filtered or centrifuged either 90 minutes after the end of the crystallization, at a crystallization temperature of 20 to 40°C, or after cooling the suspension to temperatures of 0 to 20°C. The wet product is first washed with water/acetone mixtures containing 2 to 5% of water, and finally with pure acetone. The crystalline product is dried under vacuum at 20 to 300 mbars and at 35 ~ 40°C.

The process of the invention yields crystalline sodium Cefoperazone in the form of needle microcrystals where at least 50% of the aggregates have diameter above 10 µm and at least 10% of the aggregates have diameter above 150 µm (particle size distribution was determined by laser diffraction on the dry product).

### DESCRIPTION OF THE FIGURE

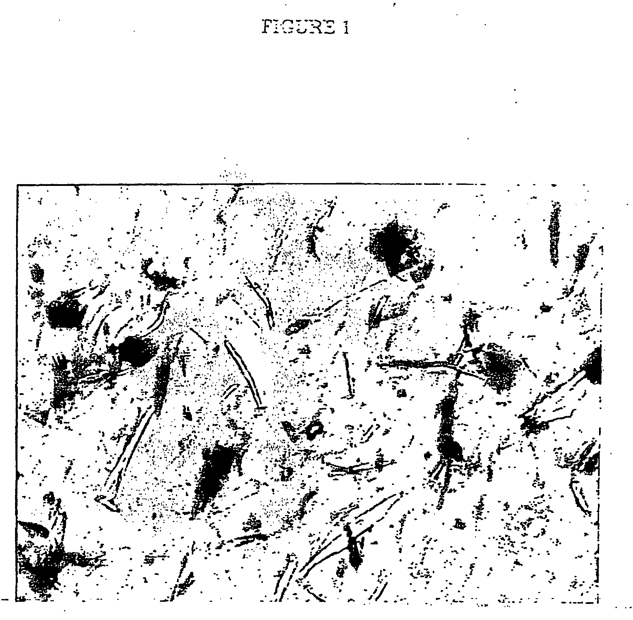

Figure 1 is an optical microscope photograph of the crystalline sodium Cefoperazone in the form of needle free microcrystals obtained from acetone without using alcohols, according to US 4,456,753 (example 2).

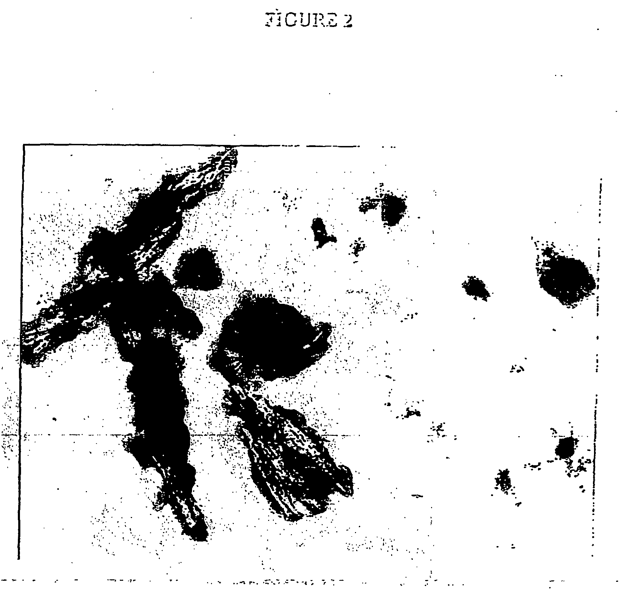

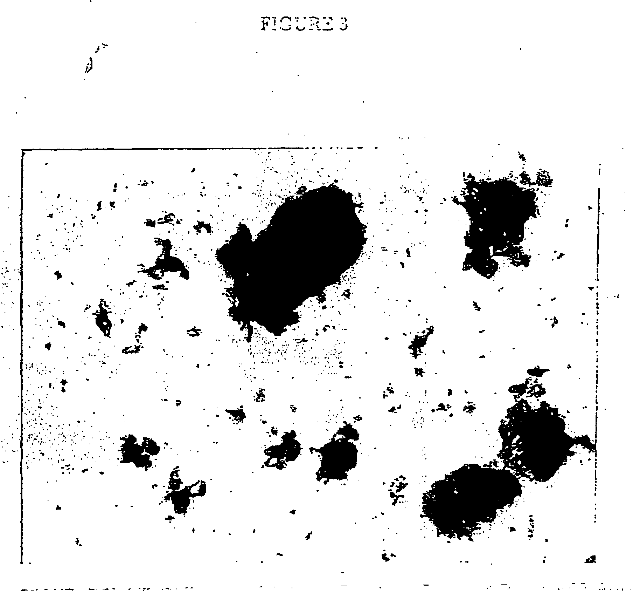

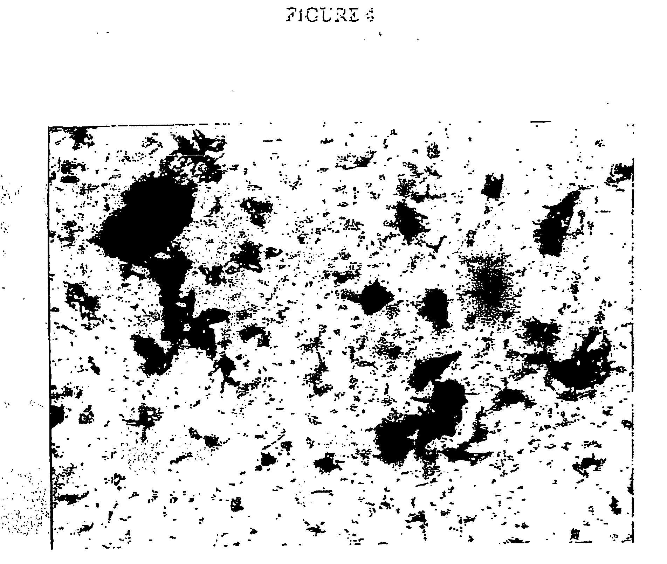

Figures 2, 3 and 4 are optical microscope photographs of the crystalline sodium Cefoperazone in the form of needle microcrystal agglomerates obtained from acetone in the presence of alcohols according to the following examples 1-3, respectively.

### EXAMPLES

The following examples illustrate the invention in greater detail.

### Example 1 - Crystallization of sodium Cefoperazone in the presence of ethanol

To a suspension of 39.45 grams of acid Cefoperazone in 118.8 ml of acetone at 20ö25°C, under vigorous stirring, a solution of 4.79 grams of sodium bicarbonate in 59.4 ml of purified water was added. The sodium Cefoperazone solution, pH 5.9ö6.0 at 25°C, was diluted with 8 ml of absolute ethanol without formation of precipitate. Acetone, 269 ml, was added under stirring at 25°C in 30 minutes until slight, but persistent turbidity. The suspension was stirred for 5 minutes at 25°C until intense turbidity, then 1013 ml of acetone were added dropwise in 20 minutes. After stirring for 60 minutes at 25°C, the product was recovered by filtration under vacuum. The crystal was washed first with a mixture of 15 ml of water and 323 ml of acetone, then with 280 ml of pure acetone. After drying for 15 hours under 1 mbar at 35°C, 36.90 grams of crystalline sodium Cefoperazone were obtained.

### Example 2 - Crystallization of sodium Cefoperazone in the presence of an ethanol/isopropanol mixture

To a suspension of 39.45 grams of acid Cefoperazone in 118.8 ml of acetone at 20ö25°C, under vigorous stirring, a solution of 4.79 grams of sodium bicarbonate in 59.4 ml of purified water. The sodium Cefoperazone solution, pH 5.9ö6.0, at 25°C was diluted first with 8 ml of absolute ethanol then with 16 ml of isopropanol without formation of precipitate. Acetone, 269 ml, was added under stirring at 25°C in 25 minutes until slight, but persistent turbidity. The suspension was stirred for 5 minutes at 25°C until intense turbidity, then 1013 ml of acetone were added dropwise in 20 minutes. After stirring for 60 minutes at 25°C the product was recovered by filtration under vacuum. The crystal was washed first with a mixture of 15 ml of water and 323 ml of acetone, then with 280 ml of pure acetone. After drying for 15 hours under 1 mbar at 35°C, 37.65 grams of crystalline sodium Cefoperazone were obtained.

### Example 3 - Crystallization of sodium Cefoperazone in the presence of isopropanol

To a suspension of 39.45 grams of acid Cefoperazone in 118.8 ml of acetone at 20ö25°C, under vigoruos stirring, a solution of 4.79 grams of sodium bicarbonate in 59.4 ml of purified water was added. The sodium Cefoperazone solution, pH 5.9ö6.0 at 25°C, was diluted with 16 ml of isopropanol without formation of precipitate. Acetone, 269 ml, was added under stirring at 25°C in 25 minutes until slight, but persistent turbidity. The suspension was stirred for 60 minutes at 25°C until intense turbidity, then 1013 ml of acetone were added dropwise in 20 minutes. After stirring for 60 minutes at 25°C the product was recovered by filtration under vacuum. The crystal was washed first with a mixture of 15 ml of water and 323 ml of acetone, then with 280 ml of pure acetone. After drying for 15 hours under 1 mbar at 35°C, 37.52 grams of crystalline sodium Cefoperazone were obtained.

## Claims

1. A process for the preparation of crystalline sodium Cefoperazone in the form of needle microcrystal aggregates, which comprises the following steps:
a) preparation of a water/acetone/sodium Cefoperazone solution containing 19 ∼ 35% of water;
b) addition of a C1-C4 aliphatic alcohol or a mixture thereof;
c) crystallisation by the addition of acetone.

2. A process according to claim 1 wherein the aliphatic alcohol is selected from ethanol, isopropanol or mixtures of ethanol and isopropanol.

3. A process according to claims 1 or 2 wherein acetone is added in two portions.

4. A process according to claim 3 wherein a first acetone portion is added to obtain a suspension containing from 11 to 14% of water and a second acetone portion is added to obtain a suspension containing from 3 to 5% of water.

5. Aggregates of crystalline sodium Cefoperazone in the form of needle microcrystals aggregates obtainable by the process of any one of claims 1-4.

6. Aggregates of crystalline sodium Cefoperazone as claimed in claim 5, where at least 50% of the aggregates have diameter above 10 µm and at least 10% of the aggregates have a diameter above 150 µm.

## Revendications

1. Procédé de préparation de Céfopérazone de sodium cristalline sous la forme d'agrégats de microcristaux aciculaires, qui comprend les étapes suivantes :
a) préparation d'une solution eau/acétone/Céfopéra-zone de sodium contenant 19-35% d'eau ;
b) addition d'un alcool aliphatique en C1-C4 ou d'un mélange de celui-ci ;
c) cristallisation par l'addition d'acétone.

2. Procédé selon la revendication 1 dans lequel l'alcool aliphatique est choisi parmi l'éthanol, l'isopropanol et des mélanges d'éthanol et d'isopropanol.

3. Procédé selon les revendications 1 ou 2 dans lequel l'acétone est ajoutée en deux portions.

4. Procédé selon la revendication 3 dans lequel une première portion d'acétone est ajoutée pour obtenir une suspension contenant 11 à 14% d'eau et une seconde portion d'acétone est ajoutée pour obtenir une suspension contenant 3 à 5% d'eau.

5. Agrégats de Céfopérazone de sodium cristalline sous la forme d'agrégats de microcristaux aciculaires, que l'on peut obtenir par le procédé de l'une quelconque des revendications 1-4.

6. Agrégats de Céfopérazone de sodium cristalline tels que revendiqués dans la revendication 5, au moins 50% des agrégats ayant un diamètre de plus de 10 µm et au moins 10% des agrégats ayant un diamètre de plus de 150 µm.

## Patentansprüche

1. Verfahren zur Herstellung von kristallinem Cefoperazon-Natrium in der Form von Nadel-Mikrokristall-Aggregaten, das die folgenden Schritte umfasst:
a) Herstellen einer Wasser/Aceton/Cefoperazon-Natrium-Lösung, die 19 ∼ 35 % Wasser enthält;
b) Zugeben eines aliphatischen C1-C4-Alkohols oder einer Mischung davon;
c) Kristallisation durch die Zugabe von Aceton.

2. Verfahren nach Anspruch 1, worin der aliphatische Alkohol ausgewählt wird aus Ethanol, Isopropanol oder Mischungen von Ethanol und Isopropanol.

3. Verfahren nach Ansprüchen 1 oder 2, worin Aceton in zwei Portionen zugegeben wird.

4. Verfahren nach Anspruch 3, worin eine erste Acetonportion zugegeben wird, um eine Suspension zu erhalten, die von 11 bis 14 % Wasser enthält, und eine zweite Acetonportion zugesetzt wird, um eine Suspension zu erhalten, die von 3 bis 5 % Wasser enthält.

5. Aggregate von kristallinem Cefoperazon-Natrium in der Form von Nadel-Mikrokristall-Aggregaten, erhältlich durch das Verfahren nach irgend einem der Ansprüche 1-4.

6. Aggregate von kristallinem Cefoperazon-Natrium nach Anspruch 5, worin mindestens 50 % der Aggregate einen Durchmesser von oberhalb 10 µm und mindestens 10 % der Aggregate einen Durchmesser von oberhalb 150 µm aufweisen.
